(19) Europäisches Patentamt
European Patent Office
Office européen des brevets

(11) EP 1 424 400 A1

(12) EUROPEAN PATENT APPLICATION

(43) Date of publication:
**02.06.2004 Bulletin 2004/23**

(51) Int Cl.[7]: **C12Q 1/68**

(21) Application number: **02292924.4**

(22) Date of filing: **26.11.2002**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(71) Applicants:
- **Arysta Lifescience Corporation**
  **Tokyo 104-6591 (JP)**
- **Patrimoine de la Faculté Universitaire des Sciences Agronomiques de Gembloux**
  **5030 Gembloux (BE)**

(72) Inventors:
- **Renaville, Robert**
  **5030 Gembloux (BE)**
- **Parmentier, Isabelle**
  **5020 Tembloux (BE)**

(74) Representative: **Desaix, Anne et al**
**Ernest Gutmann - Yves Plasseraud S.A.**
**3, rue Chauveau-Lagarde**
**75008 Paris (FR)**

# (54) Methods and kits for the selection of animals having certain milk production capabilities, based on the analysis of a polymorphism in the growth hormone receptor gene

(57) The present invention relates to a genetic marker used to distinguish amongst animals a trait for milk producing capabilities and milk composition. The marker is a mutation in the gene encoding somatotropine (also called growth hormone)-receptor (GH-R) at amino acid position 279 (a phenylalanine to tyrosine substitution in the transmembrane domain) which results in two forms of somatotropin receptor existing in bovine. The invention also pertains to methods and kits for determining whether a bovine has a genotype indicative of a trait of superior milk yield or a genotype indicative of a trait of superior milk composition, based on the analysis of said genetic marker.

EP 1 424 400 A1

SEQUENCE LISTING

<110>  ARYSTA CORPORATION
PATRIMOINE DE LA FACULTE UNIVERSITAIRE DES SCIENCES AGRONOMIQUES DE GEMBLOUX

<120>  METHODS AND KITS FOR THE SELECTION OF ANIMALS HAVING CERTAIN MILK PRODUCTION CAPABILITIES, BASED ON THE ANALYSIS OF A POLYMORPHISM IN THE SOMATOTROPIN RECEPTOR GENE

<130>  B5467 - FL/VMA/VG

<140>  New European Patent Application
<141>  2002-11-26

<160>  11

<170>  PatentIn version 3.1

<210>  1
<211>  21
<212>  DNA
<213>  OLIGONUCLEOTIDE

<220>
<221>  misc_feature
<222>  (1)..(21)
<223>  SEQ ID NO:1

<400>  1
tgggctagca gtgacattgt a                                            21

<210>  2
<211>  21
<212>  DNA
<213>  OLIGONUCLEOTIDE

<220>
<221>  misc_feature
<223>  SEQ ID NO:2

<400>  2
tgggctagca gtgacattgt t                                            21

<210>  3
<211>  21
<212>  DNA
<213>  OLIGONUCLEOTIDE

<220>
<221>  misc_feature
<222>  (1)..(21)
<223>  SEQ ID NO:3

<400>  3
gtagtcacta gcctcaccct c                                            21

<210> 4
<211> 20
<212> DNA
<213> OLIGONUCLEOTIDE

<220>
<221> misc_feature
<222> (1)..(20)
<223> SEQ ID NO:4

<400> 4
cagtgacatt atatttactc 20

<210> 5
<211> 20
<212> DNA
<213> OLIGONUCLEOTIDE

<220>
<221> misc_feature
<222> (1)..(20)
<223> SEQ ID NO:5

<400> 5
cagtgacatt atttttactc 20

<210> 6
<211> 30
<212> DNA
<213> OLIGONUCLEOTIDE

<220>
<221> misc_feature
<222> (1)..(30)
<223> SEQ ID NO:6

<400> 6
ccagtttcca tggttcttaa ttattatctt 30

<210> 7
<211> 29
<212> DNA
<213> OLIGONUCLEOTIDE

<220>
<221> misc_feature
<222> (1)..(29)
<223> SEQ ID NO:7

<400> 7
gctaaataac tggcaaaaca tatcagagt 29

<210> 8
<211> 25
<212> DNA
<213> OLIGONUCLEOTIDE

<220>
<221> misc_feature
<222> (1)..(25)
<223> SEQ ID NO:8

<400> 8
cgaatgtgtc ttgaatcctc ataca 25

<210> 9
<211> 24
<212> PRT
<213> BOVINE

<220>
<221> MISC_FEATURE
<222> (1)..(24)
<223> SEQ ID NO:9

<400> 9

```
Phe Pro Trp Phe Leu Ile Ile Ile Phe Gly Ile Leu Gly Leu Ala Val
1               5                   10                  15

Thr Leu Phe Leu Leu Ile Phe Ser
            20
```

<210> 10
<211> 24
<212> PRT
<213> BOVINE

<220>
<221> MISC_FEATURE
<222> (1)..(24)
<223> SEQ ID NO:10

<400> 10

```
Phe Pro Trp Phe Leu Ile Ile Ile Phe Gly Ile Leu Gly Leu Ala Val
1               5                   10                  15

Thr Leu Tyr Leu Leu Ile Phe Ser
            20
```

<210> 11
<211> 955
<212> DNA

```
<213>  BOVINE

<220>
<221>  misc_feature
<222>  (1)..(955)
<223>  SEQ ID NO:11


<400>  11
cttgggctag cagtgacatt atttttactc atattttcta aacagcaaag gtaagtgtga      60

tataacctac tctgatatgt tttgccagtt atttagcaaa tgtccatgtt tccatttttt     120

gtttgatgtt ttcttttgtg aatcctgagt gaagtgtttc atcaacccag tgaaacgtta     180

tcgctctaca tttacatctt tgttgtgtcc acagagagac aacacaggtc tcagttttat     240

ctggaaagtt gcataggatg ttaagagggt gaggctagtg actacatacc atgtgacatg     300

caccttaaag ttccgcactg atatttattc caggacccag aggtagcttt gagcaaaaat     360

ttaagtggtg aactaaagct actagataat tcagtctaat aaaacctttc tttagacttc     420

atatgatacc aatcttaagt aaatttgggt ttatttaaat tggttggcta cttacagttt     480

ggtattttac cttcttttgt cagagataaa attctaagtt tgaggacacc atcctgcatc     540

ctcttgcagc cagaaggcag gtttcagtta ttattctgcc actgttgttt gagttcattt     600

gagtcccttt atctctagga ctccacgttc tcatgggtaa tttgagggtg gtggattgta     660

tgatgtttaa gtttccctta agctgtaagg accattattc tattttcatt cttgaaagaa     720

actcagtata gactacagaa actttttaata caaaatactg gaaaagttac tggtgtgggt    780

tgccagtttt cttacattta gaatatgcct taagtcccat aggttaatct tgtcccttct     840

cctagaatta gggtagctaa cactccattt gcctgggtct ttcctggttt taacactaaa     900

aattctgtgt ctaggcaaac tgagacagtt ggttactcta tctaaaacca aatac          955
```

## Description

The present invention relates to the fields of dairy farming and breeding, and provides a genetic marker associated with different milk traits. More precisely, the present invention pertains to a polymorphism in the somatotropin receptor (also called *growth hormone receptor*, or GH-R) gene, which can be used as a genetic marker indicative of traits in animals such as milk production and composition.

Selection of a particular trait in a mammal is presently very expensive and very slow. Usually, the selection process involves a genealogical evaluation of the mammal's history over a long period of time. This evaluation is based on various traits of the mammal such as milk yield, protein and fat percentages in the milk, conformation traits and the like.

To date, most of the selection methods of animals exhibiting a particular trait involve visually characterizing the specific trait over a time frame or weighing the animal at particular times. The animals with the expected traits are then bred with similar animals such that the particular trait is hopefully dominant in the next generation or the generations to follow.

Hence, the present methods for trait selection in mammals are often long, tedious and open to judgement of an expert in the field, such as a breeder. For example, in order to select a good milk producing cow, it takes between 36 to 48 months to make a choice, which is often based on hypotheses and on the breeder's judgement. Moreover, there is never any real assurance that the selected trait will still dominate over the forthcoming generations.

In view of the uncertainty, expenses and time involved with the current methods of trait selection in animals, new methods are currently under development, based on a more technical approach which will hopefully improve the selection process.

Genetic improvement of livestock depends essentially on the precise estimation of the genetic value of the animals in a breeding population for a given trait. One such method is the study of candidate genes to determine whether specific genes are associated with conformational or performance traits in mammals and can therefore be used as molecular markers to select particular traits of interest. This method first requires identification of candidate genes or anonymous genetic markers associated with the traits of interest. For the candidate gene approach to be successful, genes must first be identified in the species of interest and correlated to the traits of interest using efficient quantitative genetic and statistic methods.

The somatotropin system involves several genes that may play a role in the control of particular traits in animals, since this system is associated with growth, lactation, reproduction and immunity. The administration of somatotropin, which is legally accepted in the USA, clearly indicates the role of somatotropic axis in the control of milk production in mammals. The somatotropin system is quite complicated and involves at a hypothalamic level, somatocrinin and somatostatin; at a hepatic level, somatotropin receptor and somatotropin plasmatic transport protein; and at a cellular level, somatotropin receptor, insulin-growth factor-1 and insulin growth factor transport protein. The somatotropin system is also present at a pituitary level, which is responsible for somatotropin synthesis in mammals.

The present invention is based on the selection of a gene, the somatotropin- (also called growth hormone-) receptor gene (hereinafter referred to as GH-R gene), as a genetic marker to characterize specific traits in animals, specially milk yield and composition.

The somatotropin receptor crossreacts with the somatotropin hormone to induce cells response and modulate physiological process. Hence, any mutation of this gene can alter by diminution or augmentation the hormone activities, resulting in polymorphisms which affect the outcome of different traits in an animal. Somatotropin receptor (GH-R) deficiency in cattle is associated with a miniature condition resulting in mature cattle of approximately 70% height and weight of normal size. For normal growth, growth hormone produced in the body travels in the blood and binds to the GH-R in liver and other tissues, and turns on the production of another growth regulating hormone, insulin-like growth factor-I (IGF-I).

The cDNA of the bovine transmembrane GHR has been sequenced by Hauser *et al*. (1990). It consists of 9 exons and is associated to chromosome 20 (Moody, Pomp et al. 1995). As deduced from the human nucleotide sequence, the GHR is a protein of 620 amino acids comprising an extracellular hormone binding domain of 246 amino acids, a single 24 amino acid transmembrane region and a long cytoplasmic domain. The extracellular domain contains seven cysteine residues and five potential N-linked glycosylation sites and two boxes important for the signal transduction after binding of the hormone to its receptor (Leung, Spencer et al. 1987; Wang and Wood 1995).

Using the restriction enzymes *TaqI, BgIII, DraI, EcoRV, HindIII, PstI, PvuII*, and hybridization with a heterologous rabbit GHR cDNA probe, Høj *et al*. did not observe any RFLP for Red Danish calves and Norwegian Red heifers (Hoj, Fredholm et al. 1993).

In contrast with this study and after hybridization with a homologous cDNA probe containing the coding sequence for the intracellular C-terminal part of the receptor, the inventors have identified six restriction enzyme *TaqI* bands (7.1, 6.2, 5.7, 5.4, 4.2 and 3.3 kb) that give nine genotypes in Holstein-Friesian AI bulls (Falaki, Gengler et al. 1996). The effect of this polymorphism on values for milk protein percentage was highly significant ($P < 0.005$) and favorable for the rare (6.6%) 5.7- and 5.4-kb patterns. Another interesting point of view results in the segregation of a quantitative trait locus (QTL) on chromosome 20 reported by Arranz *et al*., that affects milk yield and composition in a Holstein-Friesian dairy cattle population (Arranz, Coppieters et al. 1998).

As described in more details below, the inventors have identified a polymorphism consisting of a phenylalanine to tyrosine substitution at amino acid position 279 in the transmembrane domain of the somatotropin receptor, by direct sequencing of the corresponding DNA sequence (exon 8) of the GH-R gene from DNA of 20 different animals.

The inventors have then demonstrated that this polymorphism could be used to characterize traits such as milk production and composition in animals. Two alleles, *Phe* and *Tyr* which are responsible of three genotypes, homozygous *Phe*/*Phe*, homozygous *Tyr*/*Tyr* and heterozygous *Phe*/*Tyr*, were distinguished for the GH-R gene using an allele-specific PCR method or a Real-Time PCR method (example 1). The *Phe*/*Phe* genotype was less frequent than the *Phe*/*Tyr* or *Tyr*/*Tyr* genotypes.

The obtained data indicated that the *Tyr* allele is favourable for milk yield and unfavourable for fat and protein percentages and cell score mean while the *Phe* allele is favourable for fat and protein percentages and cell score mean and unfavourable for milk yield (example 3).

This observation allows the use of a polymorphism in the GH-R gene as a genetic marker for the identification of certain traits in animals. Once these particular traits are identified, the animals can be provided to farmers with increased value due to their superior traits.

The present invention aims at overcoming some of the disadvantages of the current methods of trait selection in animals, by providing a technical method for the selection of traits in milk production by use of a genetic marker.

Accordingly, it is an object of the present invention to provide a genetic marker for trait selection in animals. The invention also provides a process to characterize animals having superior milk production or composition traits.

Throughout this text, several words are employed, the meaning of which should be understood according to the following definitions :

As used herein, the "animal" is a bovine.

An "allele" is one of alternative forms of a genetic locus; a single allele for each locus is inherited separately from each parent. Two alleles of the same locus are responsible for three genotypes, i.e., two homozygous and one heterozygous genotypes.

The term "polymorphism" refers to a difference in DNA sequence among individuals. The simultaneous occurrence in the population of genomes showing allelic variations can be seen for example in changes in DNA affecting the restriction pattern or in alleles producing different phenotypes. The term "polymorphism" can hence be used also to designate a difference in an amino acid sequence.

The present specification discloses a polymorphism in the GH-R gene, which can have two allelic forms, depending of the nucleotide present at position 836 of its coding sequence (A or T), resulting in a variation at amino acid position 279 (Tyr or Phe). A sequence of the bovine GH-R has been described by Hauser *et al*. (1990), and can be found in GenBank under number X70041. As developed below, allele A (*Tyr*) is correlated with a higher yield of milk production, whereas allele T (*Phe*) is correlated with higher fat and protein percentages and cell score mean in milk.

As used herein, the term "trait" encompasses any characteristic, especially one that distinguishes one animal from another.

Throughout this text, the phrase "superior milk composition" will be used to qualify a milk that has a higher fat and/or protein percentage, and/or a higher cell score mean than another one. According to this definition, a milk "A" having a higher fat percentage and a higher cell score mean, and approximately the same protein content that a milk "B", is considered as having a superior composition than said milk "B".

The phrase "superior milk yield" will be used herein to express the fact that a cow is able to produce more milk, in terms of volume, than another cow. The terms "superior milk production capacities" will sometimes be used in the same meaning.

Other definitions will appear in the course of the text.

A first aspect of the present is a genetic marker for distinguishing amongst bovines those that have a trait of superior milk yield from those that have a trait of superior milk composition, wherein said marker is a polymorphism in the growth hormone receptor (GH-R) gene.

A preferred genetic marker according to this invention is a polymorphism located in exon 8 of the GH-R gene and, more preferably, a transition from T to A at nucleotide position 836 of the GH-R coding sequence, resulting in a transition from phenylalanine to tyrosine at amino acid position 279. As illustrated in example 3, the *Tyr* allele is favourable for milk production capacities (i.e., milk yield) and unfavourable for fat and protein percentages and cell score mean in milk (i.e., milk composition), and the *Phe* allele is favourable milk composition and unfavourable for milk yield.

The inventors have also demonstrated that the *Tyr* allele is dominant, since both the *Phe*/*Tyr* and *Tyr*/*Tyr* genotypes are associated with a superior milk yield, whereas the *Phe*/*Phe* genotype is associated with a superior milk composition. The genetic marker according to the invention can hence be used to quickly and easily deduce phenotypic information about the milk traits of a bovine.

Therefore, the invention also pertains to a method for determining whether a bovine has a genotype indicative of a trait of superior milk yield or a genotype indicative of superior milk composition traits, by analyzing in said bovine a genetic marker as described above. This method can be useful to determine the fate of an animal, possibly very early in its life. Indeed, different criteria are relevant to evaluate the "quality" of milk, depending on the destination of said milk. For example, a dairy farmer who wants to sell milk only for drinking will prefer cows that produce more milk in terms of quantity, and low-fat milk producing cows, whereas cheese producers may prefer protein and fat-rich milk, especially for certain kinds of cheese like parmesan. Methods and tests to determine the quality of milk that will be produced by a cow, even before its first lactation, can hence be very useful.

In one embodiment of the above method, a first step is performed to isolate genomic DNA from said bovine. This step necessitates to obtain a sample from the animal such as, but not limited to semen, blood, cells, biopsy tissues, hair bulbs and the like. Genomic DNA can then be extracted for the specimens obtained using methods known in the art as described by Sambrook et al., Molecular Cloning, A Laboratory Manual, third edition 2001. Specific techniques that can be used to isolate DNA when performing the methods according to the invention, are the protocol described in Walsh, Biotechniques, 10:506 (1991) for semen, or the protocol for blood as described by Lewin and Stewart-Haynes Biotechniques, 13:522. Kits available on sale can be also used. This step can be followed, if necessary, by a step of amplification of at least a fragment of the GH-R gene, by any method known by the skilled person, for example using standard PCR procedures, as described in Sambrook et al.,supra. If PCR is used to amplify a fragment of the GH-R gene, it is preferable, however, to amplify the genomic DNA in reaction volumes of 50 µl or less, containing 2 mM $MgCl_2$. Of course, the polymorphism analysis can also be performed on the mRNA or on the cDNA. The methods of the invention can hence comprise a step of isolating said mRNA and, if necessary, performing a reverse transcription thereof to obtain the cDNA.

In a preferred embodiment of the method according to the invention, the genetic marker is a polymorphism located in exon 8 of the GH-R gene. More preferably, this genetic marker is a transition from T (allele *Phe*) to A (allele *Tyr*) at nucleotide position 836 of the GH-R coding sequence, resulting in a transition from phenylalanine to tyrosine at amino acid position 279, as described above.

Several methods known by the skilled artisan can be used to detect the mutation in the somatotropin receptor fragment, either directly after obtaining the sample, or when necessary extracting the genomic DNA. Any detection method can be used to detect the mutation. Examples of these methods include, but are not limited to allele-specific PCR, Real-Time PCR, sequencing, SNPs and single base mutations as described by Prosser, Trends Biotech 11: 238-246 (1993) and Sambrook et al.,supra.

The present invention hence pertains to a method for determining whether a bovine has a genotype indicative of a trait of superior milk yield or a genotype indicative of a trait of superior milk composition, said method comprising the steps of :

(1) isolating nucleic acid, for example genomic DNA, from said bovine,

(2) isolating and/or amplifying a fragment of said nucleic acid, encompassing the nucleotide at position 836 of the GH-R coding sequence,

(3) determining which nucleotide is present at said nucleotide position 836 of the GH-R coding sequence, and

(4) deducing whether a bovine has a genotype indicative of a trait of superior milk yield (A/A and A/T genotypes) or a genotype indicative of a trait of superior milk composition (T/T genotype).

In preferred embodiments of the invention, the genetic marker analysis is performed using allele-specific PCR or Real-Time PCR.

When the analysis is performed using allele-specific PCR, the following primers can be used :
5'-TGGGCTAGCAGTGACATTGTA-3' (SEQ ID NO:1) specific for allele *Tyr,*
5'-TGGGCTAGCAGTGACATTGTT-3' (SEQ ID NO:2) specific for allele *Phe*, and
5'-GTAGTCACTAGCCTCACCCTC-3' (SEQ ID NO:3), as the reverse primer.

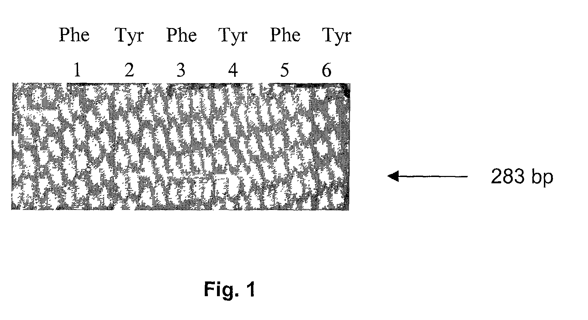

As illustrated in example 1 and Figure 1, these primers lead to the amplification of a 283 pb fragment for each of the *Tyr* or *Phe* alleles.

Alternatively the oligonucleotide 5'-CGAATGTGTCTTGAATCCTCATACA-3' (SEQ ID NO:8) can be used as a reverse primer for allele-specific amplification with the primers of SEQ ID Nos: 1 and 2.

Of course, other set of primers can be used to perform the allele-specific PCR. For example, a different reverse primer can be used with the same specific primers, or a completely set can be designed with specific primers hybridising to the other DNA strand.

When performing the genetic marker analysis by allele-specific PCR, the following conditions for the PCR reaction can be employed : between 88°C to 98°C for 10 to 15 minutes; and between 90°C to 100°C for about 1 minute, followed by between 25 to 50 cycles at between 90°C to 100°C for 20 to 40 seconds; 40°C to 60°C for 1 to 5 minutes; and 68°C to 80°C for about 1 to 5 minutes. The last step may encompass a cycle at between 68°C to 80°C for 8 to 12 minutes.

The sample can then be electrophoresed on an agarose gel to identify the amplification products with a stain such as, for example ethidium bromide, although any suitable stain can be used to identify the fragments.

As mentioned above, a particular embodiment of the method of the invention involves a genetic marker analysis performed using Real-Time PCR. This Real-Time PCR can be for example performed with the following oligonucleotides :

5'-CAGTGACATTATATTTACTC-3' (SEQ ID NO:4) specific for allele *Tyr,*
5'-CAGTGACATTATTTTTACTC-3' (SEQ ID NO:5) specific for allele *Phe,*
5' CCAGTTTCCATGGTTCTTAATTATTATCTT-3' (SEQ ID NO:6), as a forward primer, and
5'-GCTAAATAACTGGCAAAACATATCAGAGT-3' (SEQ ID NO:7), as a reverse primer.

The Real-Time PCR can be performed as described in the art. According to usual protocols, the specific oligonucleotides are differently labelled, so that the amplification of each allele is immediately detected by the device.

The present invention is not limited to the methods discussed above and encompasses any method for detecting a mutation, applied to the specific mutation identified herein.

The invention also pertains to a kit for determining whether a bovine has a genotype indicative of a trait of superior milk yield or a genotype indicative of a trait of milk composition, said kit comprising oligonucleotides to perform allele-specific amplification and/or detection of a fragment of the GH-R gene.

The kit of the invention can for example comprise the following primers :

5'-TGGGCTAGCAGTGACATTGTA-3' (SEQ ID NO:1) specific for allele *Tyr,*
5'-TGGGCTAGCAGTGACATTGTT-3' (SEQ ID NO:2) specific for allele *Phe*, and
5'-GTAGTCACTAGCCTCACCCTC-3' (SEQ ID NO:3), as a reverse primer.

Alternatively, a kit according to the invention can comprise the following oligonucleotides :

5'-CAGTGACATTATATTTACTC-3' (SEQ ID NO:4) specific for allele *Tyr,*
5'-CAGTGACATTATTTTTACTC-3' (SEQ ID NO:5) specific for allele *Phe,*
5' CCAGTTTCCATGGTTCTTAATTATTATCTT-3' (SEQ ID NO:6), as a forward primer, and
5'-GCTAAATAACTGGCAAAACATATCAGAGT-3' (SEQ ID NO:7), as a reverse primer.

Of course, any set of oligonucleotides enabling the detection of alleles *Tyr* and *Phe* can replace the sets of oligonucleotides cited above, whichever the technique used. These primers are preferably between 10 and 50 nucleotides long, and comprise more preferably between 15 and 30 nucleotides.

For performing the allele-specific amplification method, it is clear that the primers specific for each allele must encompass the nucleotide at position 836 of the GH-R coding sequence, at or near the 3' end of the primer, in order to specifically induce the amplification of a particular allele. These primers can be complementary to any of the strands of the gene, provided the reverse primer is complementary to the other strand. The skilled artisan will choose the reverse primer in such a way as to enable the amplification of a fragment of the GH-R gene — comprising the polymorphic region — with one or the other of the allele-specific primers, which implies that it will be able to anneal to the strand elongated from said allele-specific primer. The melting temperatures of the primers for the allele-specific PCR method will preferably be close to each other. Any set of primers as described above, and enabling the allele-specific amplification of a fragment of the GH-R gene, will be considered as a functional equivalent of the set of primers consisting in primers of SEQ ID Nos 1 to 3.

For performing a Real-Time PCR as described above and in the examples, the allele-specific oligonucleotides must, of course, also comprise the nucleotide at position 836 of the GH-R coding sequence, in order to specifically hybridize to a particular allele. These primers can be complementary to any of the strands of the gene, but are preferably both complementary of the same strand (i.e., the coding or non-coding one). The forward and reverse primers will be chosen by the skilled artisan in such a way as to enable the amplification of a fragment of the GH-R gene comprising the nucleotide at position 836 of the GH-R coding sequence. Any set of primers as described above, and enabling the polymorphism analysis of the GH-R gene by Real-Time PCR, will be considered as a functional equivalent of the set of primers consisting in primers of SEQ ID Nos 4 to 7.

In order to calculate the oligonucleotides' melting temperatures (Tm) and to design appropriate sets of primers for performing the methods according to the invention and for constituting the kits of the invention, the skilled person can use a variety of software such as *Primer Express* (Applied Biosystems).

The kits of the invention can also further comprise other components, such as PCR reagents, nucleic acids to be used as a control, reagents and/or columns for genomic DNA extraction, electrophoresis gels, dyes, and the like. Of course, the kits can comprise only part of the elements cited above.

The content of the kits may vary depending upon the detection methods utilized, and the skilled artisan will adapt the kits according to the method to be used and possibly to the user.

In order to further illustrate the present invention and advantages thereof, the following specific examples are given, it being understood that they are intended only as illustrative and in nowise limitative.

**BRIEF DESCRIPTION OF THE DRAWINGS**

Fig. 1 is an electrophoretic gel illustrating the Allele-specific PCR patterns using the specific primers on the somatotropin receptor gene observed in 3 Holstein-Friesian bulls. Lanes 1 and 2 show the result of *Phe*- and *Tyr*-allele specific amplification from an homozygous *Tyr* bull, Lanes 3 and 4 for an heterozygous one, and lanes 5 and 6 for an homozygous *Phe* bull.

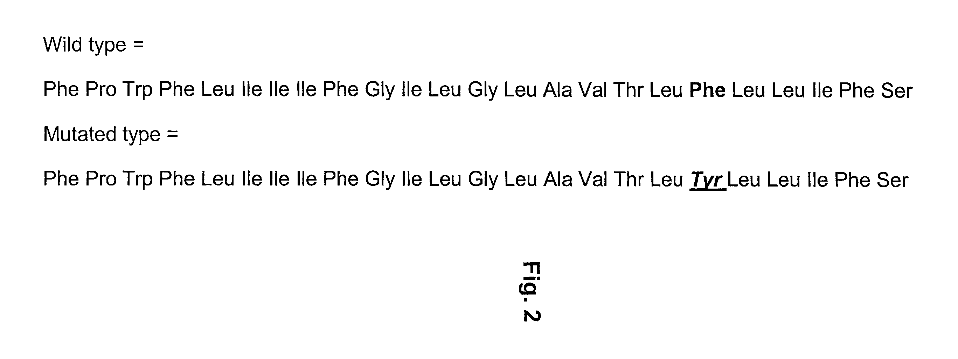

Fig. 2 shows the amino acid sequence obtained after sequencing of the transmembrane domain of the somatotropin receptor gene in bovine species (SEQ ID Nos: 9 and 10). The amino acid affected by the mutation is in bold.

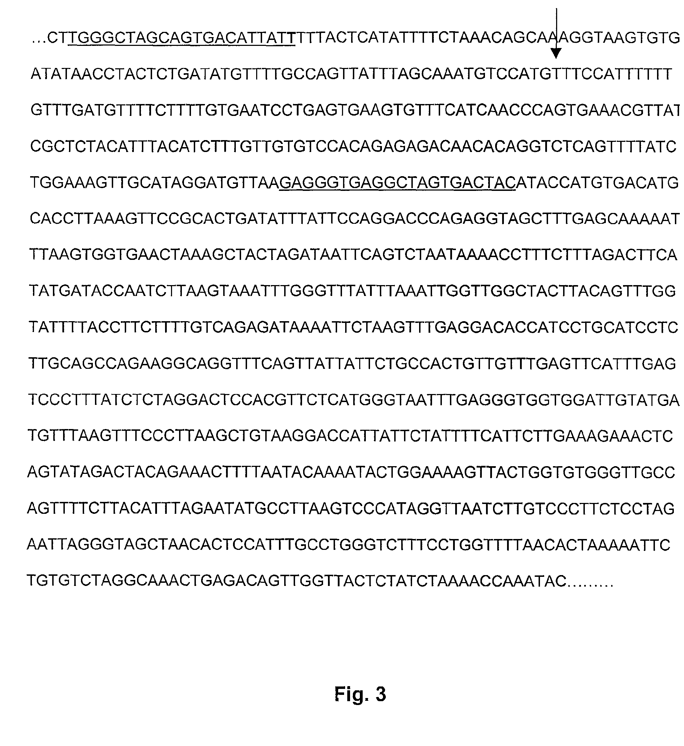

Fig. 3 shows the nucleotide sequence obtained by sequencing the region downstream exon 8 of the GH-R gene (SEQ ID NO:11). The primers used for allele-specific amplification are underlined in the sequence. The nucleotide at the polymorphic locus is in bold. The vertical arrow indicates the frontier between the end of exon 8 and the beginning of intron 8.

**EXAMPLES**

***1. DNA EXTRACTION AND PCR***

Genomic DNA of 1100 commercially available registered Canadian Holstein-Friesian bulls was extracted from semen as described by Lucy et al., Domest. Anim. Endocrinol. 10:325 (1993).

The mutation at transmemebrane domain (exon 8) of the somatotropin receptor gene was revealed by allele-specific PCR or Real-Time PCR analysis.

A. The allele-specific PCR method is the following: The specific PCR primers for phenylalanine (T) allele and tyrosine (A) alleles were designed from exon 8 of the somatotropin receptor gene. The sequences of the primers used were 5'TGGGCTAGCAGTGACATTGTA-3' as a primer specific for allele *Tyr* (SEQ ID No. 1 ), 5'-TGGGCTAGCAGTGACATTGTT-3' as a primer specific for allele *Phe* (SEQ ID No. 2), and 5'-GTAGTCACTAGCCTCACCCTC-3' as a reverse primer (SEQ ID No. 3).
These primers were used to amplify by standard procedures fragments of the genomic DNA in 25-µl reaction volumes containing 2 mM MgCl2. Conditions were 96°C, 3 min. followed by 35 cycles of 94°C, 1 min, 65.2°C, 1 min., and 72°C, 1 min. The last step was 72°C for 10 min. PCR products (283 bp) were electrophoresed on 2% agarose gels with 1 µg/ml ethidium bromide (Figure 1).

B. The Real-Time PCR specific method : This technology combines thermal cycling, fluorescence detection, and application-specific software in a single instrument. Specific oligonucleotides with MGB fluorescent have been designed to specifically identify allele *Tyr* or allele *Phe* in a single detection multiplex. In this method, each allele is immediately detected by the device (in the present case, the 7700 model of Applied Biosystem (other devices can be used)). The following oligonucleotides have been used :
5'-CAGTGACATTATATTTACTC-3' primer specific for allele *Tyr* (SEQ ID No. 4), labelled with FAM
5'-CAGTGACATTATTTTTACTC-3' primer specific for allele *Phe* (SEQ ID No. 5), labelled with VIC
5'-CCAGTTTCCATGGTTCTTAATTATTATCTT-3' forward primer (SEQ ID No. 6) (unlabelled)
5'-GCTAAATAACTGGCAAAACATATCAGAGT-3' reverse primer (SEQ ID No. 7) (unlabelled).
These primers were used to amplify by standard Real Time procedures part of the genomic DNA in 5 µl reaction volumes containing 2.5 µl of "Taqman Universal PCR Master Mix3" (ref. 430443). Conditions were 50°C, 2 min and 95°C, min. followed by 40 cycles of 95°C, 15 sec, 60°C, 1 min. PCR products were vizualized by 7900 Real-Time PCR Apllied Biosystem device.

***2. ANIMALS***

Test-day data were provided by Canadian Dairy Network for the Holstein breed and were the same data that were used in the genetic evaluation in Canada in May 2000. In this study only first lactation data were analysed. These data included 12,858,741 test-day records for 1,656,599 cows in production born between 1982 and 1999. The structure of the population represents the current Canadian Holstein population. The mean production and standard-deviation per cow per day were 24.56±6.4 kg for milk, 903±237 g for fat, 792±192 g for protein and 5.79±1.76 for somatic cell score (somatic cell score = $\log_2$(SCS), wherein SCS is the amount of somatic cells measured in the milk (comprising cell debris, normal cells, bacteria, etc.)). A pedigree file was extracted from Holstein Canadian database and included 2,755,041 animals (cows and ancestors) born between 1909 and 1999. Semen samples of 961 Canadian Holstein bulls were provided by Semex Alliance

***3. STATISTICAL ANALYSIS***

**3. A. Model**

An alternative test day model to the official one was used. This analysis model provided flexibility for the fixed portion and a minimum number of parameters for the random portion through the use of polynomials. Random regression effects were modelled using modified Legendre polynomials, in order to reduce correlations among regression coefficients. The use of third order polynomials (constant, linear and quadratic) was sufficient for a single yield trait to describe the random variation around the fixed lactation curve (Gengler et al., 1999). The three modified Legendre polynomials used were:

$$l_0 = 1$$

$$l_1 = \sqrt{3}x$$

$$l_2 = \sqrt{5/4}(3x^2-1)$$

where:

$$x = -1 + 2\ ((DIM -1) / (305 -1))$$

and DIM = days in milk

The model used for the estimation of the different effects in the first lactation test-day records was :

$$y = q_1a_1 + q_2a_2 + Hhtd + Ssarc + W(Zp + Z^*a) + e$$

where:

**y** = vector of production data (test-day yields) — 12,858,741 levels

$a_1$ = regression effect of the first allele — one level

$a_2$ = regression effect of the second allele — one level

**htd** = vector of herd and test-day fixed effects — 1,320,824 levels

**sarc =** vector of season, group of age, region and class of lactation fixed effects — 560 levels

There were two calving seasons : September to March and April to August. Four groups of calving age were defined : first <25 months, second between 25-30 months, third between 30-35 and last > 35 months. The region was a province or a group of provinces. For the ten different provinces the following regions were defined: British Colombia (region 1), Alberta (region 2), Saskatchewan (region 2), Manitoba (region 2), New found land (region 3), Ontario (region 5), Quebec (region 4), New Brunswick (region 3), Nova Scotia (region 3) and Prince Edward Island (region 3). Fourteen lactation stage classes were created. These groups corresponded at a group of 20 DIM from day 25 (<25, 45, 65...) until day 305.

**p =** vector of permanent environmental random effects — 1,656,599 levels

**a** = vector of genetic random effects — 2,755,058 levels

**e** = vector of residual effects

**H, S, Z et Z*** = incidence matrices

**$q_1$ =** vector of estimated allele frequencies of the first allele

**$q_2$ =** vector of estimated allele frequencies of the second allele

**W** = covariate matrix for the Legendre polynomials.

Regressions on both alleles were kept in the model as allele frequencies were estimated and therefore the sum of those frequencies could have been different from 1). Also it must be acknowledged that there was no adjustment for the fact that allelic frequencies were themselves estimated and therefore we observed a lost of variation in the estimated compared to the observed allele frequencies.

The estimation of the allelic frequencies is described below. A preconditioned conjugate gradient (PCG) solver was used to solve the analysis model. The allelic substitution effect was defined as the phenotypic difference obtained by substituting a copy of the second allele by a copy of the first one. This effect was estimated as:

$$\tfrac{1}{2}(a_2 - a_1)$$

where:

$a_1$ = solution for the effect of the first allele

$a_2$ = solution for the effect of the second allele

**3.B. Results**

***Genotype frequencies***

Using both of the two recommended mutation detection methods, the frequencies of the genotype in 1100 Holstein bulls was 58.69% for the *Phe*/*Phe* pattern, 34.86% for the *Tyr*/*Phe* pattern and 6.45 % for the *Tyr*/*Tyr* pattern.

The frequencies of the *Tyr* and *Phe* alleles were estimated by a maximum likelihood approach with 23.88% for *Tyr* and 76.12% for *Phe*.

***Relationship of phenylalanine to tyrosine susbstitution to Milk production***

The association of somatotropin receptor polymorphism and milk traits in dairy cattle was shown on the alternative test day model.

Estimated production effects of a substitution of a copy of the second allele (*Phe*) by a copy of the first one (*Tyr*) are

+ 295 g/day for milk,
- 8.14 g/day for fat,
- 1.83 g/day for protein and
- 0.22/day for cell score.

In term of mean production, the effects of a substitution of a copy of the second allele (*Phe*) by a copy of the first one (*Tyr*) are

+ 1.11% of milk mean production,
- 0.95% of fat mean production,
- 0.19% of protein mean production and
- 1.7% for cell score mean.

These results indicated that *Tyr* allele is favourable for milk yield and unfavourable for fat and protein percentages and cell score mean while *Phe* allele is favourable for milk composition and unfavourable for milk yield.

### 3.C. Conclusions

Two alleles were distinguished for the somatotropin receptor gene using an allele-specific PCR or a Real-Time PCR method. The polymorphism was observed at amino acid position 279, and consisted of a phenylalanine to tyrosine substitution in the transmembrane domain (exon 8) of the somatotropin receptor gene.

The *Phe*/*Phe* pattern was less frequent than the *Tyr*/*Phe* or *Tyr*/*Tyr* pattern.

The superiority of the GH-R *Tyr* allele was observed for milk yield while the superiority of the GH-R *Phe* allele was observed for protein and fat percentage and cell score.

Interesting enough, these findings show the usefulness of the alternative test day model to distinguish between effects on related traits.

### REFERENCES

Arranz, J. J., W. Coppieters, et al. (1998). "A QTL affecting milk yield and composition maps to bovine chromosome 20: a confirmation." Anim Genet **29**(2): 107-15.

Falaki, M., N. Gengler, et al. (1996). "Relationships of polymorphisms for growth hormone and growth hormone receptor genes with milk production traits for Italian Holstein- Friesian bulls." J Dairy Sci **79**(8): 1446-53.

Hauser, S. D., M. F. McGrath, et al. (1990). "Cloning and in vivo expression of bovine growth hormone receptor mRNA." Mol Cell Endocrinol **72**(3): 187-200.

Hoj, S., M. Fredholm, et al. (1993). "Growth hormone gene polymorphism associated with selection for milk fat production in lines of cattle." Anim Genet **24**(2): 91-5.

Leung, D. W., S. A. Spencer, et al. (1987). "Growth hormone receptor and serum binding protein: purification, cloning and expression." Nature **330**(6148): 537-43.

Moody, D. E., D. Pomp, et al. (1995). "Assignment of the growth hormone receptor gene to bovine chromosome 20 using linkage analysis and somatic cell mapping." Anim Genet **26**(5): 341-3.

Wang, Y. D. and W. I. Wood (1995). "Amino acids of the human growth hormone receptor that are required for proliferation and Jak-STAT signaling." Mol Endocrinol **9**(3): 303-11.

## Claims

1. A genetic marker for distinguishing amongst animals those that have a trait of superior milk yield from those that have a trait of superior milk composition, wherein said marker is a polymorphism in the growth hormone receptor (GH-R) gene.

2. The genetic marker according to claim 1, wherein the polymorphism is located in exon 8 of the GH-R gene.

3. The genetic marker according to claim 2, wherein the polymorphism is a transition from T (allele *Phe*) to A (allele *Tyr*) at nucleotide position 836 of the GH-R coding sequence, resulting in a transition from phenylalanine to tyrosine at amino acid position 279, wherein the *Tyr* allele is favourable for milk yield and unfavourable for milk composition and the *Phe* allele is favourable for milk composition and unfavourable for milk yield.

4. The genetic marker according to claims 2 and 3, wherein the *Phe*/*Phe* genotype is associated with a superior milk composition, and the *Phe*/*Tyr* and *Tyr*/*Tyr* genotypes are associated with a superior milk yield.

**5.** A method for determining whether a bovine has a genotype indicative of a trait of superior milk yield or a genotype indicative of a trait of superior milk composition, said method comprising the step of analyzing in said bovine a genetic marker according to any of claims 1 to 4.

**6.** The method of claim 5, comprising a first step consisting of isolating genomic DNA from said bovine.

**7.** The method of claim 5 or claim 6, wherein said genetic marker is a polymorphism located in exon 8 of the GH-R gene.

**8.** The method of any of claims 5 to 7, wherein said genetic marker is a transition from T (allele *Phe*) to A (allele *Tyr*) at nucleotide position 836 of the GH-R coding sequence, resulting in a transition from phenylalanine to tyrosine at amino acid position 279, wherein the *Tyr* allele is favourable for milk yield and unfavourable for milk composition and the *Phe* allele is favourable for milk composition and unfavourable for milk yield.

**9.** The method of any of claims 5 to 8, wherein said genetic marker analysis is performed using allele-specific PCR or Real-Time PCR.

**10.** The method of any of claims 5 to 9, wherein said genetic marker analysis is performed using allele-specific PCR with the following primers :
5'-TGGGCTAGCAGTGACATTGTA-3' (SEQ ID NO:1) specific for allele *Tyr*,
5'-TGGGCTAGCAGTGACATTGTT-3' (SEQ ID NO:2) specific for allele *Phe*, and
5'-GTAGTCACTAGCCTCACCCTC-3' (SEQ ID NO:3), as a reverse primer,
or equivalents thereof.

**11.** The method of any of claims 5 to 9, wherein said genetic marker analysis is performed using Real-Time PCR with the following oligonucleotides :
5'-CAGTGACATTATATTTACTC-3' (SEQ ID NO:4) specific for allele *Tyr,*
5'-CAGTGACATTATTTTTACTC-3' (SEQ ID NO:5) specific for allele *Phe,*
5' CCAGTTTCCATGGTTCTTAATTATTATCTT-3' (SEQ ID NO:6), as a forward primer, and
5'-GCTAAATAACTGGCAAAACATATCAGAGT-3' (SEQ ID NO:7), as a reverse primer,
or equivalents thereof.

**12.** A kit for determining whether a bovine has a genotype indicative of a trait of superior milk yield or a genotype indicative of a trait of superior milk composition, said kit comprising oligonucleotides to perform allele-specific amplification and/or detection of a fragment of the GH-R gene.

**13.** The kit of claim 12, wherein the oligonucleotides enable the amplification and/or detection of a fragment of tge GH-R gene encompassing the nucleotide at position 836 of the GH-R coding sequence.

**14.** The kit according to claim 12, which comprises the following primers :
5'-TGGGCTAGCAGTGACATTGTA-3' (SEQ ID NO:1) specific for allele *Tyr*,
5'-TGGGCTAGCAGTGACATTGTT-3' (SEQ ID NO:2) specific for allele *Phe,* and
5'-GTAGTCACTAGCCTCACCCTC-3' (SEQ ID NO:3), as a reverse primer,
or equivalents thereof.

**15.** The kit according to claim 12, which comprises the following oligonucleotides :
5'-CAGTGACATTATATTTACTC-3' (SEQ ID NO:4) specific for allele *Tyr,*
5'-CAGTGACATTATTTTTACTC-3' (SEQ ID NO:5) specific for allele *Phe,*
5' CCAGTTTCCATGGTTCTTAATTATTATCTT-3' (SEQ ID NO:6) as a forward primer, and
5'-GCTAAATAACTGGCAAAACATATCAGAGT-3' (SEQ ID NO:7), as a reverse primer,
or equivalents thereof.

**16.** The kit according to any of claims 12 to 15, further comprising PCR reagents and/or control nucleic acids and/or materials for genomic DNA extraction and/or electrophoresis gels and/or dyes.

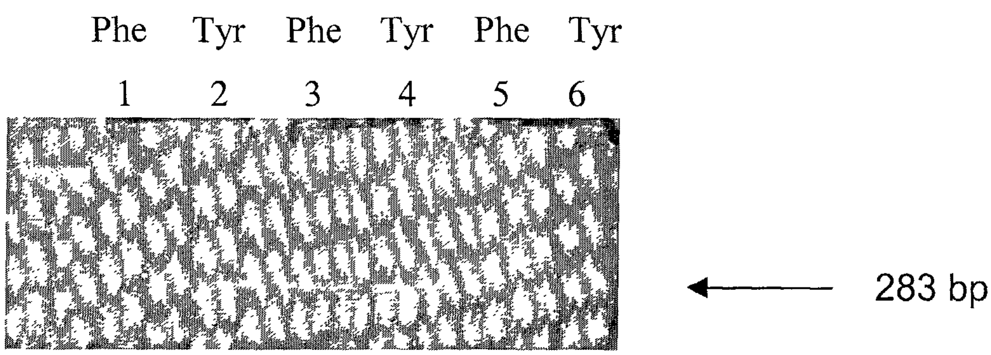


**Fig. 1**

Wild type =

Phe Pro Trp Phe Leu Ile Ile Ile Phe Gly Ile Leu Gly Leu Ala Val Thr Leu **Phe** Leu Leu Ile Phe Ser

Mutated type =

Phe Pro Trp Phe Leu Ile Ile Ile Phe Gly Ile Leu Gly Leu Ala Val Thr Leu ***<u>Tyr</u>*** Leu Leu Ile Phe Ser

Fig. 2

...CTTGGGCTAGCAGTGACATTAT**T**TTTACTCATATTTTCTAAACAGCAAAGGTAAGTGTG
ATATAACCTACTCTGATATGTTTTGCCAGTTATTTAGCAAATGTCCATGTTTCCATTTTTT
GTTTGATGTTTTCTTTTGTGAATCCTGAGTGAAGTGTTTCATCAACCCAGTGAAACGTTAT
CGCTCTACATTTACATCTTTGTTGTGTCCACAGAGAGACAACACAGGTCTCAGTTTTATC
TGGAAAGTTGCATAGGATGTTAAGAGGGTGAGGCTAGTGACTACATACCATGTGACATG
CACCTTAAAGTTCCGCACTGATATTTATTCCAGGACCCAGAGGTAGCTTTGAGCAAAAAT
TTAAGTGGTGAACTAAAGCTACTAGATAATTCAGTCTAATAAAACCTTTCTTTAGACTTCA
TATGATACCAATCTTAAGTAAATTTGGGTTTATTTAAATTGGTTGGCTACTTACAGTTTGG
TATTTTACCTTCTTTTGTCAGAGATAAAATTCTAAGTTTGAGGACACCATCCTGCATCCTC
TTGCAGCCAGAAGGCAGGTTTCAGTTATTATTCTGCCACTGTTGTTTGAGTTCATTTGAG
TCCCTTTATCTCTAGGACTCCACGTTCTCATGGGTAATTTGAGGGTGGTGGATTGTATGA
TGTTTAAGTTTCCCTTAAGCTGTAAGGACCATTATTCTATTTTCATTCTTGAAAGAAACTC
AGTATAGACTACAGAAACTTTTAATACAAAATACTGGAAAAGTTACTGGTGTGGGTTGCC
AGTTTTCTTACATTTAGAATATGCCTTAAGTCCCATAGGTTAATCTTGTCCCTTCTCCTAG
AATTAGGGTAGCTAACACTCCATTTGCCTGGGTCTTTCCTGGTTTTAACACTAAAAATTC
TGTGTCTAGGCAAACTGAGACAGTTGGTTACTCTATCTAAAACCAAATAC.........

**Fig. 3**

European Patent Office

# EUROPEAN SEARCH REPORT

Application Number
EP 02 29 2924

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| X | MOISIO S ET AL: "Polymorphism within the 3' flanking region of the bovine growth hormone receptor gene." ANIMAL GENETICS, vol. 29, no. 1, February 1998 (1998-02), pages 55-57, XP002242811 ISSN: 0268-9146 * the whole document * --- | 1,5,6, 12,16 | C12Q1/68 |
| D,X | DATABASE BIOSIS 'Online! BIOSCIENCES INFORMATION SERVICE, PHILADELPHIA, PA, US; January 1999 (1999-01) AGGREY S E ET AL: "Markers within the regulatory region of the growth hormone receptor gene and their association with milk-related traits in Holsteins." Database accession no. PREV199900126423 XP002242348 * abstract * & JOURNAL OF HEREDITY, vol. 90, no. 1, January 1999 (1999-01), pages 148-151, ISSN: 0022-1503 --- -/-- | 1,5,6,12 | TECHNICAL FIELDS SEARCHED (Int.Cl.7)<br>C12Q |

The present search report has been drawn up for all claims

| Place of search | Date of completion of the search | Examiner |
|---|---|---|
| THE HAGUE | 28 May 2003 | Gabriels, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03 82 (P04C01)

European Patent Office

# EUROPEAN SEARCH REPORT

Application Number
EP 02 29 2924

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | DATABASE BIOSIS 'Online! BIOSCIENCES INFORMATION SERVICE, PHILADELPHIA, PA, US; 1996 FALAKI M ET AL: "Relationships of polymorphisms for growth hormone and growth hormone receptor genes with milk production traits for Italian Holstein-Friesian bulls." Database accession no. PREV199699194367 XP002242349 * abstract * & JOURNAL OF DAIRY SCIENCE, vol. 79, no. 8, 1996, pages 1446-1453, ISSN: 0022-0302 ----- | 1,5,6,12 | |
| | | | TECHNICAL FIELDS SEARCHED (Int.Cl.7) |

The present search report has been drawn up for all claims

| Place of search | Date of completion of the search | Examiner |
|---|---|---|
| THE HAGUE | 28 May 2003 | Gabriels, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)